# EUROPEAN PATENT APPLICATION

(11) **EP 4 588 682 A1**
(43) Date of publication of application: **23.07.2025**
(21) Application number: 24223560.4
(22) Date of filing: 27.12.2024
(51) Int. Cl.: B41J 13/10, B41J 29/02, B41J 29/13

(54) **LINKAGE APPARATUS FOR PRINTER, PRINTER, AND MEDICAL APPARATUS**

(30) Priority: 19.01.2024 CN 202410082569
(71) Applicant: GE Precision Healthcare LLC, Waukesha, WI 53188 (US)
(72) Inventor: LU, Yi, Waukesha, 53188 (US); FAN, Li, Waukesha, 53188 (US); SHEN, Kai, Waukesha, 53188 (US); ZHANG, Long, Waukesha, 53188 (US); ZHANG, Haibo, Waukesha, 53188 (US)
(74) Representative: Fennell, Gareth Charles

(57) **Abstract**

Embodiments of the present application provide a linkage apparatus for a printer, a printer, and a medical apparatus. The linkage apparatus comprises: a paper compartment assembly, provided with a side opening, a base, a paper tray located in the base, and a paper advancing member disposed opposite the side opening, an end of the paper advancing member opposite the side opening being provided with an advancing plate extending upward; a door assembly, movably connected to the paper compartment assembly and capable of closing or opening the side opening; and a transmission mechanism, one side of which is connected to the door assembly, the transmission mechanism driving the paper advancing member to move toward the side opening along with opening of the door assembly, causing the paper advancing member to, by means of the advancing plate, advance paper in the paper tray to move toward the door assembly. Thus, usability of the printer can be improved.

## Description

### TECHNICAL FIELD

The present application relates to the field of printers, and in particular relates to a linkage apparatus for a printer, a printer, and a medical apparatus.

### BACKGROUND

In daily life, during processing of various types of services, terminal devices are each equipped with a printer used to print related bills and documents for clients to keep and use. In addition, due to the fact that portability and miniaturization are required in many use scenarios, the volumes of printers are continuously decreasing.

Terminals of some medical devices are also provided with printers. For example, for an electrocardiograph detection device, once an electrocardiograph report is printed, a user needs to remove paper from a paper tray of a printer, and if paper runs out or the printer is out of order for maintenance, paper needs to be replaced in the paper tray.

It should be noted that the above introduction of the background is only for the convenience of clearly and completely describing the technical solutions of the present application, and for the convenience of understanding for those skilled in the art.

### SUMMARY

It has been found by the inventors that the areas of openings of doors in some existing printers for printing bills and reports are excessively small, resulting in difficulties in removing paper from paper trays by users. In addition, sharp metal members or plastic members are provided at the openings of printers, such as electrocardiograph report printers, causing users to easily scratch their fingers when removing paper from narrow spaces.

In view of the above problems, in some existing printers, paper is pulled out from paper trays by means of flexible spacers placed under the paper. However, the flexible spacers have complex installation and use means, are fragile, and are apt to being rolled into the printers, causing paper jams, which can therefore affect the use convenience of the printers.

To solve the above problems and other similar problems, the embodiments of the present application provide a linkage apparatus for a printer, to make paper output and paper replacement of the printer more convenient.

According to a first aspect of the embodiments of the present application, a linkage apparatus for a printer is provided. The linkage apparatus comprise:
a paper compartment assembly, provided with a side opening, a base, a paper tray located in the base, and a paper advancing member disposed opposite the side opening, the end of the paper advancing member opposite the side opening being provided with an advancing plate extending upward;
a door assembly, movably connected to the paper compartment assembly and capable of closing or opening the side opening; and
a transmission mechanism, one side of which is connected to the door assembly, the transmission mechanism driving the paper advancing member to move toward the side opening along with opening of the door assembly, causing the paper advancing member to, by means of the advancing plate, advance paper in the paper tray to move toward the door assembly.

According to a second aspect of the embodiments of the present application, the transmission mechanism comprises:
a gear transmission part, disposed in the base and provided with a gear set, a propeller shaft, and a rack linked to the gear set by means of the propeller shaft,
wherein at least one gear in the gear set is connected to the door assembly, and, along with opening and closing movements of the door assembly, the gear set drives the propeller shaft to rotate, thus driving the rack to move in the base, the rack being linked with the paper advancing member, and when the rack moves in the base, the paper advancing member accordingly moving in the base.

According to a third aspect of the embodiments of the present application, one end and the other end of the propeller shaft are provided with a first gear and a second gear, respectively, the first gear being connected to the gear set, and the second gear being connected to the rack.

According to a fourth aspect of the embodiments of the present application, the rack is provided with a step section, an extending section, and a connecting section,
wherein the step section meshes with the propeller shaft, the extending section extends from one end of the step section in a direction toward the paper advancing member, the connecting section extends from one end of the extending section in a direction toward the paper advancing member and is connected to the paper advancing member, and the width of the connecting section is less than that of the extending section.

According to a fifth aspect of the embodiments of the present application, the linkage apparatus further includes a rotation lock part, disposed in the base, one end of which is connected to the transmission mechanism, and the other end of which is connected to the paper advancing member so as to be openable and closable, and the transmission mechanism, by means of the rotation lock part, drives the paper advancing member to move.

According to a sixth aspect of the embodiments of the present application, the rotation lock part is provided with: a shaft, a rotation arm, a torsion spring and a rotation lock; and
the rotation arm, the torsion spring and the rotation lock are fastened to the other side of the transmission mechanism by means of the shaft, and are rotatable about the shaft, so as to implement rotatable connection with the transmission mechanism.

According to a seventh aspect of the embodiments of the present application, the paper advancing member is further provided with a locking part, and a paper receiving part connected to the locking part, the advancing plate protruding upward from the paper receiving part; and
the rotation lock engages with the locking part, so as to connect the rotation lock part and the paper advancing member.

According to an eighth aspect of the embodiments of the present application, the rotation lock is provided with an engagement part protruding downward from an extending end thereof;
the locking part is provided with a recess section recessed downward, and the engagement part engages with the recess section; and
an extending end of the locking part and an extending end of the engagement part of the rotation lock each have slopes whose shapes fit together.

According to a ninth aspect of the embodiments of the present application, the linkage apparatus further includes a boss provided on the base; the rotation arm of the rotation lock part, after moving a particular distance in a direction toward the side opening, is capable of abutting against the boss and rotating, so as to release engagement between the rotation lock and the locking part.

According to a tenth aspect of the embodiments of the present application, a hollow structure is provided between the transmission mechanism and the base, and the boss is accommodated in the hollow structure.

According to an eleventh aspect of the embodiments of the present application, the linkage apparatus further includes a limiting part, the limiting part being disposed at the end of the side opening of the base, and the limiting part being capable of preventing the door assembly from opening farther when the door assembly is opened a particular amount.

According to a twelfth aspect of the embodiments of the present application, the paper tray comprises: a top cover and a bottom cover; and
the bottom cover is disposed on the base, and a space for accommodating paper is formed between the top cover and the bottom cover.

According to a thirteenth aspect of the embodiments of the present application, the bottom cover is provided with an opening, and the opening has a shape matching that of the advancing plate of the paper advancing member, enabling the advancing plate of the paper advancing member to move in the opening.

According to a fourteenth aspect of the embodiments of the present application, a printer is provided, the printer being provided with the linkage apparatus according to the first aspect to the twelfth aspect.

According to a fifteenth aspect of the embodiments of the present application, a medical apparatus is provided, the medical apparatus being provided with the printer according to the thirteenth aspect.

According to a sixteenth aspect of the embodiments of the present application, the medical apparatus comprises an electrocardiograph machine.

One of the beneficial effects of the embodiments of the present application is that: according to the linkage apparatus for a printer provided in the embodiments of the present application, when a user uses a printer equipped with the linkage apparatus provided in the embodiments of the present application, movement of the door assembly can be transferred to the paper only by rotating the rotatable door assembly, and once the user opens the door assembly, the paper is accordingly advanced by the paper advancing member, by means of said action, to a position close to the side opening. Therefore, the user can easily remove paper from or put paper into the paper tray, thereby improving the usability of the printer.

With reference to the following description and drawings, specific implementations of the present application are disclosed in detail, and the means by which the principles of the present application can be employed are illustrated. It should be understood that the embodiments of the present application are not limited in scope thereby. Within the scope of the spirit and clauses of the appended claims, the embodiments of the present application include many changes, modifications, and equivalents.

The features described and/or illustrated for one implementation may be used in one or more other implementations in the same or similar manner, be combined with features in other embodiments, or replace features in other implementations.

It should be emphasized that the terms "include/comprise" when used herein refer to the presence of features, integrated components, steps, or assemblies, but do not preclude the presence or addition of one or more other features, integrated components, steps, or assemblies.

### BRIEF DESCRIPTION OF THE DRAWINGS

The included drawings are used to provide further understanding of the embodiments of the present application, which constitute a part of the description and are used to illustrate the implementations of the present application and explain the principles of the present application together with textual description. Evidently, the drawings in the following description are merely some embodiments of the present application, and a person of ordinary skill in the art may obtain other drawings according to the drawings without the exercise of inventive effort. In the drawings:
FIG. 1 is a cross-sectional view of a linkage apparatus for a printer according to an embodiment of the present application;
FIG. 2 is a cross-sectional view of a transmission mechanism in the linkage apparatus according to an embodiment of the present application;
FIG. 3 is a top view of the linkage apparatus from an upper-side perspective according to an embodiment of the present application;
FIG. 4 is a schematic diagram of a rack of the transmission mechanism in the linkage apparatus according to an embodiment of the present application;
FIG. 5 is a schematic diagram of the linkage apparatus according to an embodiment of the present application;
FIG. 6 is a schematic diagram of components of a rotation lock in the linkage apparatus according to an embodiment of the present application;
FIG. 7 is a cross-sectional view of the linkage apparatus in which a rotation lock part is connected to the rack according to an embodiment of the present application;
FIG. 8 is a schematic diagram of the linkage apparatus in which the rotation lock is engaged with a paper advancing member according to an embodiment of the present application;
FIG. 9 is a schematic diagram of states of the rotation lock in the linkage apparatus according to an embodiment of the present application;
FIG. 10 is a schematic diagram of components of the linkage apparatus for the printer according to an embodiment of the present application;
FIG. 11 is another schematic diagram of components of the linkage apparatus for the printer according to an embodiment of the present application.
FIG. 12 is a flowchart of a paper removal process of a printer equipped with the linkage apparatus according to an embodiment of the present application;
FIG. 13 is a flowchart of a paper placement process of the printer equipped with the linkage apparatus according to an embodiment of the present application;
FIG. 14 is a flowchart of a door closing process of the printer equipped with the linkage apparatus according to an embodiment of the present application; and
FIG. 15 is a schematic diagram of an electrocardiograph machine equipped with the linkage apparatus according to an embodiment of the present application.

### DETAILED DESCRIPTION

The foregoing and other features of the embodiments of the present application will become apparent from the following description with reference to the drawings. In the description and drawings, specific implementations of the present application are disclosed in detail, and a portion of the implementations in which the principles of the present application may be employed are explicated. It should be understood that the present application is not limited to the described implementations, and on the contrary, includes all modifications, variations, and equivalents which fall within the scope of the appended claims.

In the embodiments of the present application, the term "and/or" includes any and all combinations of one or more associated listed terms. The terms "comprise", "include", "have", etc., refer to the presence of described features, elements, components, or assemblies, but do not exclude the presence or addition of one or more other features, elements, components, or assemblies.

In the embodiments of the present application, the singular forms "a" and "the", etc., may include plural forms, and should be broadly construed as "a type of" or "a class of," rather than being limited to the meaning of "one". Furthermore, the term "the" should be construed as including both the singular and plural forms, unless otherwise specified in the context. In addition, the term "according to" should be construed as "at least in part according to..." and the term "on the basis of' should be construed as "at least in part on the basis of...", unless otherwise specified in the context.

Implementations of the embodiments of the present application are described below with reference to the accompanying drawings.

Embodiments of the present application provide a linkage apparatus for a printer.

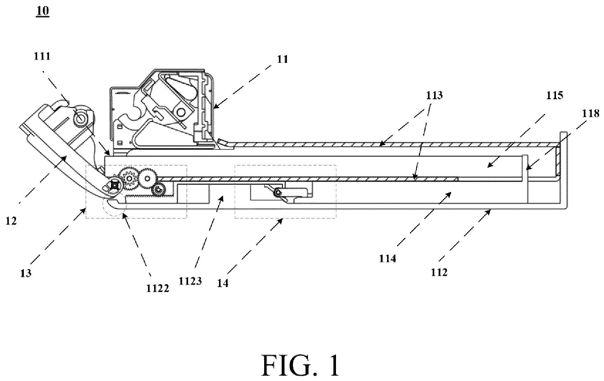

FIG. 1 is a cross-sectional view of a linkage apparatus 10 for a printer according to an embodiment of the present application. As shown in FIG. 1, the linkage apparatus 10 according to the embodiments of the present application comprises: a paper compartment assembly 11, a door assembly 12, and a transmission mechanism 13.

The paper compartment assembly 11 is provided with a side opening 111, a base 112, a paper tray 113 located in the base 112, and a paper advancing member 114 disposed opposite the side opening 111. The end of the paper advancing member 114 opposite the side opening 111 (the right end shown in FIG. 1) is provided with an advancing plate 118 extending upward. Paper 115 is accommodated in the paper compartment assembly 11.

The door assembly 12 is movably connected to the paper compartment assembly 11, and is used to open or close the side opening 111.

The transmission mechanism 13 is disposed in the base 112 at a position near the side opening 111. A side of the transmission mechanism 13 facing toward the side opening 111 is connected to the door assembly 12. The transmission mechanism 13 can drive the paper advancing member 114 to move toward the side opening 111 along with opening of the door assembly 12, causing the paper advancing member 114 to, by means of the advancing plate 118, advance the paper 115 in the paper tray 113 to move toward the door assembly 12.

By means of the above structure, when a user uses the printer equipped with the linkage apparatus 10 provided in the embodiments of the present application, movement of the door assembly 12 can be transferred to the paper 115 in the paper tray 113 simply by operating the rotatable door assembly 12, e.g., by pressing the door assembly 12, and once the user opens the door assembly 12, the paper 115 is accordingly advanced by the advancing plate 118 of the paper advancing member 114, by means of said action, out to a position close to the side opening 111. Therefore, the user can easily remove paper from or put paper into the paper tray, thereby improving the usability of the printer.

In the embodiments of the present application, the door assembly 12 may be movably connected to the paper compartment assembly 11 by various means. In some embodiments, the door assembly 12 is mounted on the base 112 and configured to be rotatable within a particular range about a connection with the base 112; during use, when a user applies an external force to rotate the door assembly 12 into contact with the paper compartment assembly 11, the door assembly 12 can engage with the paper compartment assembly 11 by means of an engagement unit such as a door lock, to thereby close the side opening 111. The door assembly 12 can further be separated from the paper compartment assembly 11 by an external force applied by a user, thereby opening the side opening 111.

In some embodiments, the door assembly 12 may be engaged and locked with the paper compartment assembly 11 by means of a door lock. In addition, the door assembly 12 may alternatively be locked with the paper compartment assembly 11 by means of other structures, such as a torsion spring and an interference fit. This is not limited in the present application, reference may be made to related technologies in the art, and details are not described herein.

In some embodiments, to restrict a movement range of the transmission mechanism 13 and the paper advancing member 114 in the base 112, the base 112 of the linkage apparatus 10 of the embodiments of the present application is further provided with a limiting part 1122. The limiting part 1122 may be disposed at an end of the side opening 111 of the base 112, and when the door assembly 12 is opened a particular amount, the limiting part 1122 can prevent the door assembly 12 from opening farther, so as to prevent the transmission mechanism 13 from being moved farther out of the side opening 111. In this way, the transmission mechanism 13 can be prevented from being pulled out of the base 112.

In some embodiments, the transmission mechanism is implemented by means of gear transmission. The present application is not limited thereto, and unless otherwise specified in the present application, the transmission mechanism may alternatively be implemented by means of linking rod transmission and/or belt transmission, which are known in the art, as long as opening and closing actions of the door assembly 12 can be transferred to the paper advancing member 114. In the following description, description is provided using a transmission mechanism implemented by means of gear transmission as an example.

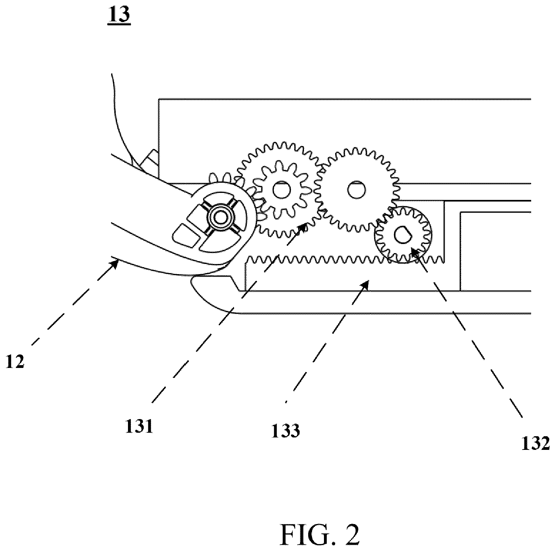

FIG. 2 is a cross-sectional view of the transmission mechanism 13 in the linkage apparatus 10 according to an embodiment of the present application. As shown in FIG. 1 and FIG. 2, the transmission mechanism 13 includes a gear transmission part disposed in the base 112, the gear transmission part being provided with a gear set 131, a propeller shaft 132, and a rack 133 linked to the gear set 131 by means of the propeller shaft 132.

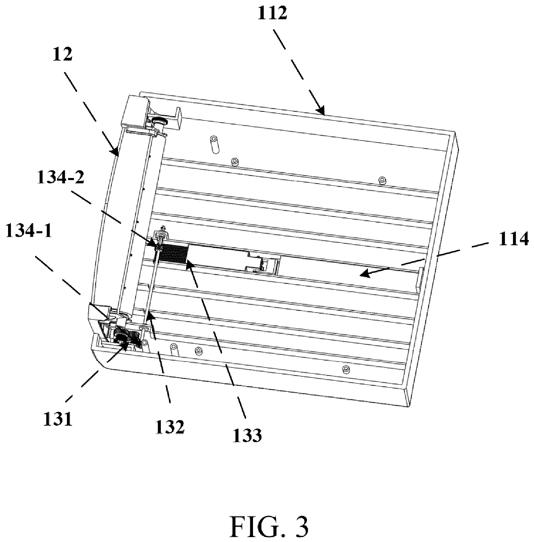

FIG. 3 is a top view of the linkage apparatus 10 from an upper-side perspective according to an embodiment of the present application. As shown in FIG. 2 and FIG. 3, at least one gear in the gear set 131 is connected to the door assembly 12, and along with opening and closing movements of the door assembly 12, the gear set 131 further drives the propeller shaft 132 to rotate, so that the propeller shaft 132 can thereby drive the rack 133 to move in the base 112. Specifically, as shown in FIG. 3, one end and the other end of the propeller shaft 132 are provided with a first gear 134-1 and a second gear 134-2, respectively, one end of the propeller shaft 132 meshing with the gear set 131 by means of the first gear 134-1, and the other end of the propeller shaft 132 meshing with the rack 133 by means of the second gear 134-2. Therefore, during rotation of the gear set 131 along with the opening and closing movements of the door assembly 12, the propeller shaft 132 rotates together with the gear set 131 by means of the first gear 134-1, and further transfers the rotation to the rack 133 by means of the second gear 134-2, thereby driving the rack 133 to move in the base 112.

By means of the described structure, the rack 133 can be linked with the paper advancing member 114, and during movement of the rack 133 in the base, this pushes the paper advancing member 114 to move in the base 112. Therefore, the paper advancing member 114 can advance the paper 115 out of the side opening 111 by means of the advancing plate 118.

In addition, as shown in FIG. 3, the gear set 131 is disposed at the edge of the base 112, the rack 133 and the paper advancing member 114 are disposed at positions close to the middle position inside the base 112, and the gear set 131 and the rack 133 are connected and driven by means of the propeller shaft 132. Therefore, the structure of the transmission mechanism 13 is more flexible, and the advancing plate 118 of the paper advancing member 114 can advance the paper more stably at the middle position.

In the above embodiments, as shown in FIG. 1 and FIG. 2, for example, the gear set 131 includes three gears. The present application is not limited thereto. For example, only one connecting structure is provided for the gear set 131. In a specific application, different gear set connecting structures may be adopted according to different printer dimensions. For example, one gear is used to form the gear set 131, or more than three gears are used to form the gear set 131, and so on. In addition, in the above embodiments, the length of the propeller shaft 132 is set according to the position of the rack 133, and in a specific implementation, may alternatively be adjusted according to the dimensions of a printer and/or the position of the rack 133, which is not limited in the present application. Reference may be made to related technologies in the related art, and details are not described herein.

In the above embodiments, as shown in FIG. 3, for example, the propeller shaft 132 meshes with and is connected to the gear set 131 and the rack 133 by means of two gears (the first gear 134-1 and the second gear 134-2), respectively. The present application is not limited thereto. The propeller shaft 132 may alternatively be connected to the gear set 131 and the rack 133, respectively, by means of other structures, e.g., by means of a linking rod and/or a transmission belt, as long as the rotation of the gear set 131 can be transferred to the rack 133, causing the rack 133 to drive the paper advancing member 114 to move.

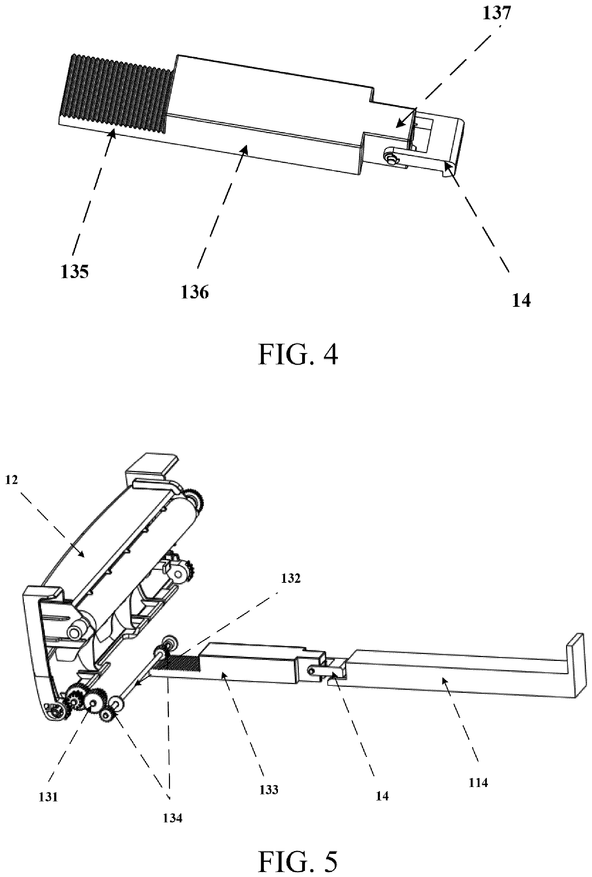

FIG. 4 is a schematic diagram of the rack 133 of the transmission mechanism 13 in the linkage apparatus 10 according to an embodiment of the present application. As shown in FIG. 4, in some embodiments, the rack 133 is provided with a step section 135, an extending section 136, and a connecting section 137.

In the above embodiment, the step section 135 meshes with the second gear 134-2 of the propeller shaft 132, the extending section 136 extends from one end of the step section 135 (the right end of the step section 135 shown in FIG. 4) in a direction toward the paper advancing member 114, the connecting section 137 extends from one end of the extending section 136 (the right end of the extending section 136 shown in FIG. 4) in a direction toward the paper advancing member 114 and is connected to the paper advancing member 114, and the width of the connecting section 137 is less than that of the extending section 136. This configuration is such that, during connection between the connecting section 137 and the paper advancing member 114, parts disposed at positions along the width of the connecting section 137 do not interfere with some positioning structures, such as a shutter, disposed in the base 112, enabling the rack 133 to more smoothly drive the paper advancing member 114 to slide in the base 112.

In the above embodiments, for example, the part for connecting the paper advancing member 114 is connected on both the left and right sides in the width direction of the connecting section 137. In other embodiments, the part for connecting the paper advancing member 114 is connected to the connecting section 137 by other means. Therefore, the width of the connecting section 137 may be equal to that of the extending section 136, as long as the connection between the connecting section 137 and the paper advancing member 114 is not affected.

FIG. 5 is a schematic diagram of the linkage apparatus 10 according to an embodiment of the present application. As shown in FIG. 5, in some embodiments, a rotation lock part 14 is further provided at a connection position between the rack 133 and the paper advancing member 114. The rotation lock part 14 is configured such that one end thereof is connected to the transmission mechanism 13, e.g., the connecting section 137 of the rack 133 of the transmission mechanism 13, and the other end thereof is connected to the paper advancing member 114 so as to be openable and closable.

In the above embodiments, the transmission mechanism 13 drives the paper advancing member 114 to move by means of the rotation lock part 14.

Specifically, the transmission mechanism 13 transfers the opening and closing movements of the door assembly 12 to the rotation lock part 14, causing the rotation lock part 14 to be connected to or separated from the paper advancing member 114, thereby implementing movement of the paper advancing member 114. FIG. 5 illustrates a state in which the rack 133 is connected to the paper advancing member 114. The processes of connecting and separating the rotation lock part 14 and the paper advancing member 114 will be described below.

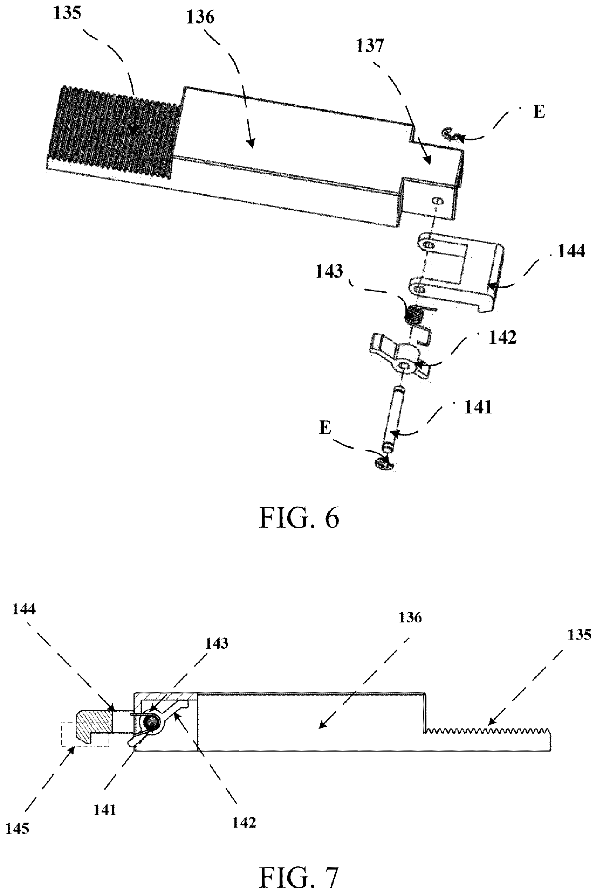

FIG. 6 is a schematic diagram of components of the rotation lock part 14 in the linkage apparatus 10 according to an embodiment of the present application. As shown in FIG. 6, in some embodiments, the rotation lock part 14 is provided with: a shaft 141, a rotation arm 142, a torsion spring 143 and a rotation lock 144.

In the above embodiment, the rotation arm 142, the torsion spring 143 and the rotation lock 144 are fastened to the other side of the transmission mechanism 13 by means of the shaft 141, and are rotatable about the shaft 141, so as to implement a rotatable connection with the transmission mechanism 13.

FIG. 7 is a cross-sectional view of the linkage apparatus 10 in which the rotation lock part 14 is connected to the rack 133 according to an embodiment of the present application. As shown in FIG. 6 and FIG. 7, in some embodiments, a through hole passing through the connecting section 137 is provided in the width direction of the connecting section 137 of the rack 133, the rotation lock 144 is provided with an opening, and the width of the opening is slightly greater than that of the connecting section 137, so that the connecting section 137 can be snap-fit into the opening of the rotation lock 144, and the shaft 141 passes through the rotation lock 144 and the through hole of the connecting section 137, to fasten the rotation lock 144 and the connecting section 137.

In the above embodiments, as shown in FIG. 7, the shaft 141 in the opening of the rotation lock 144 is further provided with a torsion spring 143 and a rotation arm 142, and two sides of the shaft 141 (outside the opening of the rotation lock 144) are each provided with an E-shaped ring E, to fasten the rotation lock part 14 onto the connecting section 137 of the rack 133 so as to be rotatable about the shaft 141.

In the above embodiments, a D-shaped shaft is used as the shaft 141 to connect the components. The present application is not limited thereto. For example, another nonconventional cylindrical shaft may alternatively be used as the shaft 141, or a cylindrical shaft may alternatively be used to perform fastening by welding.

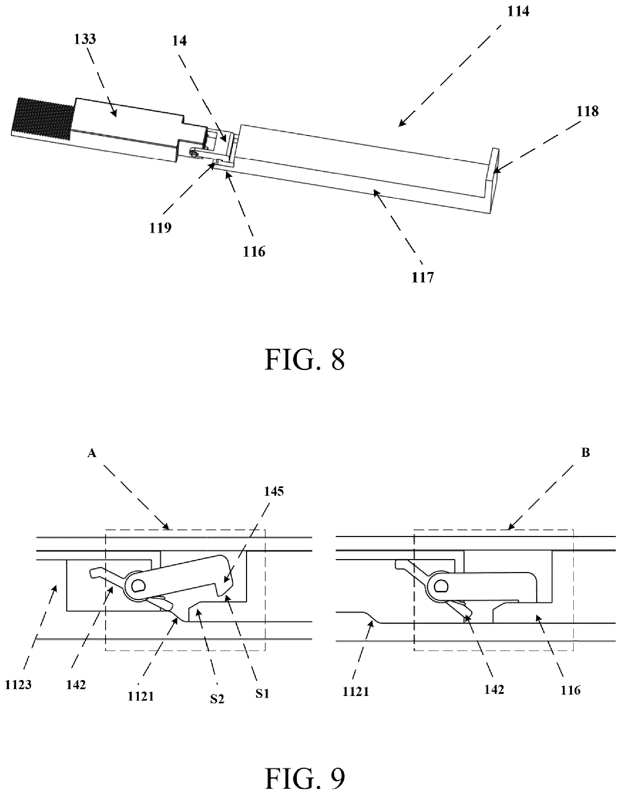

FIG. 8 is a schematic diagram of the linkage apparatus 10 in which the rotation lock part 14 engages with the paper advancing member 114 according to an embodiment of the present application. As shown in FIG. 8, in some embodiments, the paper advancing member 114 is further provided with a locking part 116, and a paper receiving part 117 having one end connected to the locking part 116, and the advancing plate 118 protrudes upward from the other end (the right end shown in FIG. 8) of the paper receiving part 117.

In the above embodiment, the rotation lock 144 engages with the locking part 116, so as to connect the rotation lock part 14 and the paper advancing member 114.

In one possible implementation, as shown in FIG. 7 and FIG. 8, the rotation lock 144 is provided with an engagement part 145 protruding downward from an extending end thereof, and the locking part 116 is provided with a recess section 119 recessed downward, so that, by means of the described structure, during connection between the rotation lock 144 and the locking part 116, the engagement part 145 engages with the recess section 119. Therefore, locking and separating between the rotation lock 144 and the locking part 116 are implemented.

FIG. 9 is a schematic diagram of states of the rotation lock 144 in the linkage apparatus 10 according to an embodiment of the present application. As shown in FIG. 9, the rotation lock 144 and the locking part 116 may be in a separated state A and a locked state B.

In one possible implementation, as shown in FIG. 9, the extending end of the locking part 116 and the extending end of the engagement part 145 of the rotation lock 144 are provided with slopes S1 and S2, respectively, having shapes which fit together.

In the above embodiment, as shown in FIG. 9, due to the fact that the rotation lock part 14 is fastened to the rack 133, during a process of the rotation lock 144 of the rotation lock part 14 moving along with the rack 133, the rotation lock 144 may move toward or away from the paper advancing member 114, and when the rotation lock 144 comes into contact with the paper advancing member 114, the slope S2 at the end of the engagement part 145 comes into contact with the slope S1 at the end of the locking part 116, the engagement part 145 slides toward the paper advancing member 114 along the slope by the force of the movement of the rack 133, and the engagement part 145 slides over the slope S1 of the locking part 116 and engages with the recess section 119, thereby achieving the locked state B between the rotation lock 144 and the locking part 116.

In the above embodiment, as shown in FIG. 9, the linkage apparatus 10 further includes a boss 1121 provided on the base 112. The rotation arm 142 of the rotation lock part 14, after moving a particular distance in a direction toward the side opening 111, is capable of abutting against the boss 1121 and rotating, so as to release engagement between the rotation lock 144 and the locking part 116, thereby achieving the separated state A between the rotation lock 144 and the locking part 116.

In the above embodiment, as shown in FIG. 9, the boss 1121 is configured to be a slope projecting in a direction away from the paper advancing member 114, but the present application is not limited thereto, and the boss 1121 may be, for example, a protrusion having an inclined angle.

In the above embodiment, as shown in FIG. 1 and FIG. 9, in one possible implementation, a hollow structure is disposed between the transmission mechanism 13 and the base 112, and the boss 1121 is accommodated in the hollow structure. The present application is not limited thereto, and the boss 1121 may alternatively be disposed on the base 112 by other means.

In the above embodiment, during movement of the rack 133 toward the side opening 111, when one end of the rotation arm 142 abuts against the boss 1121, the angle of inclination of the rotation arm 142 changes, thereby providing a force for the torsion spring 143, so that the torsion spring 143 deforms, and the torsion spring 143 thus provides a force for the rotation lock 144, so that the end of the rotation lock 144 connected to the paper advancing member 114 is lifted, thereby separating the engagement part 145 from the recess section 119, and releasing engagement between the rotation lock 144 and the locking part 116.

In some embodiments, as shown in FIG. 1 and FIG. 9, the base 112 is further provided with a protrusion 1123, the protrusion 1123 being capable of holding the extending section 136 of the rack 133, and in particular holding the extending section 136 of the rack 133 between the boss 1121 of the base 112 and the paper tray 113, so that the rack 133 can stably move in the base 112.

Therefore, because of the force provided by the opening and closing movements of the door assembly 12, the transmission mechanism 13 and the paper advancing member 114 can be separated and connected, to further drive the paper advancing member 114 to move by means of the transmission mechanism 13, so as to advance the paper 115 to move back and forth, thereby implementing paper removal and paper placement. The structure is simple, and paper removal can be facilitated during use of the printer.

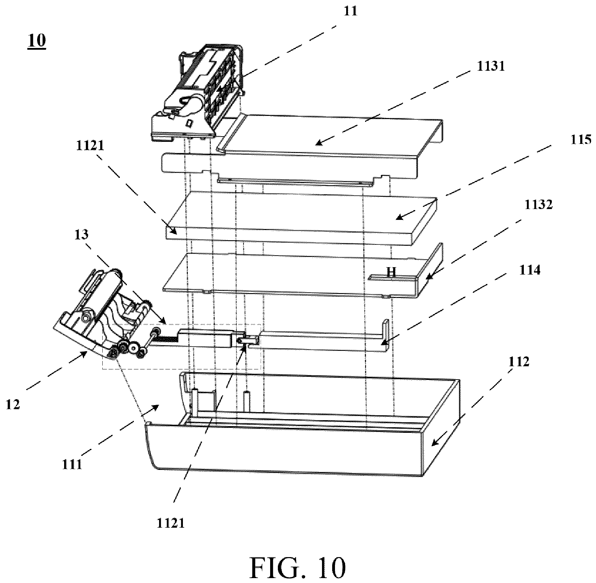

FIG. 10 is a schematic diagram of components of the linkage apparatus 10 for the printer according to an embodiment of the present application. As shown in FIG. 10, in some embodiments, the paper tray 113 includes: a top cover 1131 and a bottom cover 1132. The top cover 1131 is fastened to the base 112, to form an accommodating space with the base 112, the bottom cover 1132 is disposed in the base 112, and the paper advancing member 114 is located between the base 112 and the bottom cover 1132 of the paper tray 113.

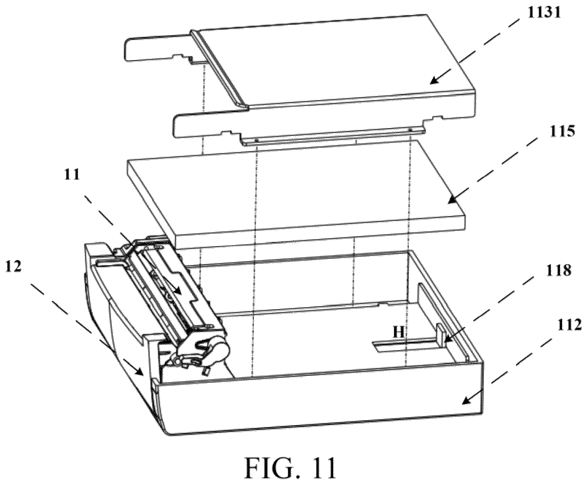

FIG. 11 is another schematic diagram of components of the linkage apparatus 10 for the printer according to an embodiment of the present application. As shown in FIG. 10 and FIG. 11, in some embodiments, the bottom cover 1132 of the paper tray 113 is provided with an opening H. The shape of the opening H matches that of the advancing plate 118 of the paper advancing member 114, e.g., the width of the opening H matches that of the advancing plate 118, so that the advancing plate 118 can move in the opening H.

In the above embodiment, the length of the opening H is not limited, and may be set according to the distance by which the paper 115 is to be advanced. Furthermore, in the above embodiment, the height of the advancing plate 118 is not limited, as long as the height is lower than that of the base 112, thereby avoiding interference with the top cover 1131.

By means of the linkage apparatus 10 provided in the above embodiments of the present application, the movement of the door assembly 12 can be transferred to the paper 115 in the paper tray 113 just by rotating the rotatable door assembly 12, and once the user opens the door assembly 12, the paper 115 is accordingly advanced by the paper advancing member 114, by means of the action, to a position close to the side opening 111. Therefore, the user can easily remove paper from or put paper into the paper tray, thereby improving the usability of the printer.

In addition, by means of the linkage apparatus 10 provided in the above embodiments of the present application, during paper removal by the user, the paper 115 is partially advanced out of the side opening 111 by the paper advancing member 114; therefore the user does not need to stick their hand into the side opening 111 to remove the paper, thereby avoiding the risk of a sharp metal member or plastic member disposed at the side opening 111 of the paper compartment assembly scratching a finger.

In addition, a large portion of the transmission mechanism of the linkage apparatus 10 provided in the present application is hidden between the bottom cover 1132 of the paper tray 113 and the base 112. The structure is simple, and the paper removal and paper placement processes can be implemented without exchanging additional parts. The operation is simple and the use is more convenient.

Next, a flowchart of using the linkage apparatus according to an embodiment of the present application will be described.

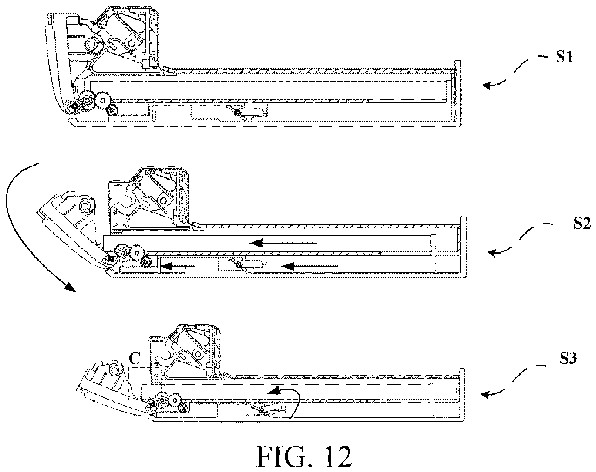

FIG. 12 is a flowchart of a paper removal process of a printer equipped with the linkage apparatus 10 according to an embodiment of the present application. As shown in FIG. 12, in the paper removal process:
In a state S1, the door assembly is initially in a completely closed state, and the door assembly is engaged with the paper compartment assembly. In this case, the linkage apparatus is in a stationary state, the rotation lock is engaged with the paper advancing member, and the rotation lock part and the paper advancing member are in a connected state.

In a state S2, the linkage apparatus is in a door opening process. During opening of the door, the gear of the door assembly drives the gear set of the transmission mechanism to rotate, and a gear of the gear set meshing with the propeller shaft drives the propeller shaft to rotate, driving the rack to move in the direction indicated by the arrow in FIG. 12, i.e., the direction toward the door assembly. The rack further drives the shaft of the rotation lock part, together with the rotation lock connected to the shaft, to move in the direction toward the door assembly. In this process, the rotation lock and the paper advancing member are in a connected state, and the rotation lock is engaged with the locking part. Therefore, the paper advancing member also moves in the direction toward the door assembly along with the rack, for the advancing plate of the paper advancing member to advance the paper to move together. In this case, the paper advancing member can advance the paper out.

In a state S3, the door assembly is opened to the maximum extent.

As shown in FIG. 12, the base is further provided with a limiting part. The limiting part may be disposed at the end of the side opening of the base. When the door assembly is opened a particular amount, the limiting part can prevent the door assembly from opening farther, so as to prevent the transmission mechanism from moving farther out of the side opening. In this way, excessive rotation of the door can be avoided, and the transmission mechanism can be prevented from being pulled out of the base.

That is, the position at which the door assembly is opened to the maximum extent is a position at which the rack abuts against the limiting part. In this case, the rotation arm abuts against the boss provided on the base, the rotation arm is rotated, and the rotation arm and the rotation lock are fastened by means of a shaft D and rotate together such that they remain still relative to each other. Therefore, the rotation lock is rotated with the movement of the rack. In this case, the rotation lock is rotated according to the arrow shown in the figure, to be further separated from the paper advancing member, and the paper advancing member and the paper stop moving outward.

However, at this time, the end of the paper close to the side opening is at the position C shown in FIG. 12, i.e., at this time, the paper has been advanced out of the side opening. Therefore, the user can remove the paper from the position C without sticking a hand into the side opening.

By means of the above paper removal process, a user can remove paper more conveniently when using a printer equipped with the linkage apparatus according to the embodiments of the present application, and the risk of finger scratching is also avoided.

When using the printer, when paper in the paper tray is used up and paper needs to be placed in the paper tray of the printer, the paper can also be placed in the paper tray more conveniently by means of the linkage apparatus provided in the embodiments of the present application. The paper placement process will be described below.

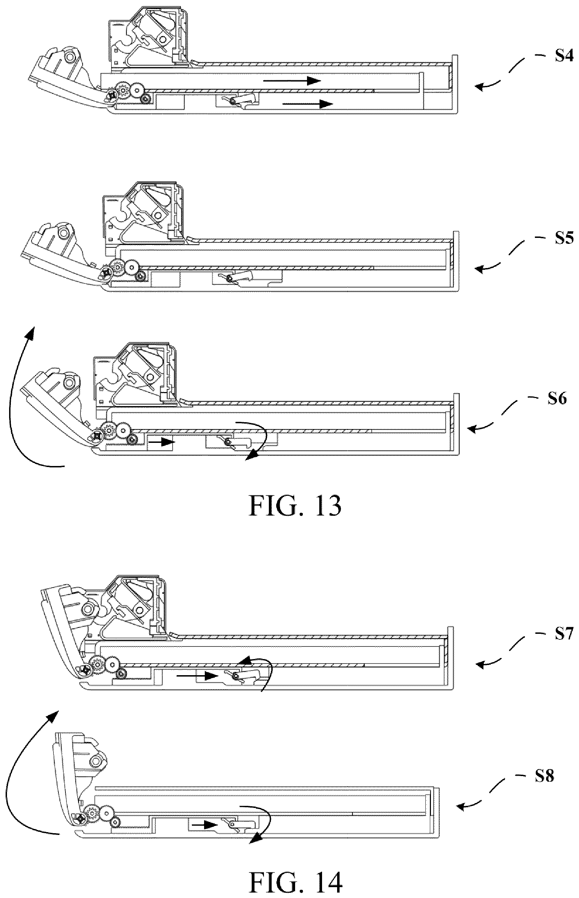

FIG. 13 is a flowchart of a paper placement process of the printer equipped with the linkage apparatus 10 according to an embodiment of the present application. As shown in FIG. 13, in the paper placement process:
In a state S4, the printer and the linkage apparatus are in a state in which a user is ready to put paper to the paper tray.

As shown in FIG. 13, the initial state of the printer is a state in which the door assembly is opened to the maximum extent, and due to the fact that the rotation lock and the advancing plate are in an unlocked and separated state at this time, when the user puts the paper into the paper tray, the paper can only be advanced using the paper advancing member, i.e., the paper and the paper advancing member are advanced back to a state in which the advancing plate of the paper advancing member abuts against the base, and the door assembly is not driven to close.

In a state S5, when the paper is completely placed by the user, as shown in FIG. 13, both the paper and the paper advancing member are advanced back to the innermost side of the base, but the door assembly is still in a state of being opened to the maximum extent at this time.

It should be noted that, at this time, the rotation arm still remains in a state of abutting against the boss of the base. Therefore, the rotation lock also remains in a lifted state.

In a state S6, the door assembly is ready to be closed after the paper is placed.

After the paper placement is completed, the door assembly is ready to be closed, and the gear on the door assembly drives the gear set to rotate, to then drive the rack, the rotation arm and the rotation lock to move toward the base. The rack starts moving inward, and the rotation arm accordingly starts sliding downward along the slope of the boss. The height of a sliding track at the bottom of the rotation arm suddenly decreases, and the rotation arm therefore rotates counterclockwise, i.e., in the direction of the arrow shown in FIG. 13, under the action of the torsion spring. At this time, the rotation lock resets to a horizontal state.

According to the above paper placement process, when the user uses the printer equipped with the linkage apparatus provided in the embodiments of the present application, the paper can be conveniently placed into the paper tray without installing an additional part, such as a paper film.

In actual operation, when the printer does not need to be used, for example, after paper removal or paper placement operations are completed, or the printer is powered off, a door closing operation needs to be performed.

FIG. 14 is a flowchart of a door closing process of the printer equipped with the linkage apparatus 10 according to an embodiment of the present application. As shown in FIG. 14, in a door closing process:
In a state S7, the door assembly is in a state immediately before closing.

In this case, the door assembly, the transmission mechanism and the rotation lock continuously move to the right, i.e., in the direction indicated by the right arrow in FIG. 14. At this time, the paper advancing member remains at the innermost side of the base. In addition, the ends of both the engagement part of the rotation lock and the locking part of the paper advancing member are provided in advance with slopes having shapes which fit together; therefore, when the rotation lock comes into contact with the paper advancing member, the rotation lock first rotates counterclockwise, i.e., rotates in the direction indicated by the curved arrow in FIG. 14. At this time, the rotation lock is slightly lifted up, causing the engagement part of the rotation lock to move further inward.

In a state S8, the door assembly is in a completely closed state.

When the door assembly is completely closed, the door assembly is engaged with the paper compartment assembly, the side opening is closed, and the engagement part of the rotation lock and the locking part of the paper advancing member further slide to the tail of the slopes; that is, during the inward movement of the rotation lock, the slopes of the engagement part and the locking part slide to positions where the engagement part and the locking part are no longer in contact with each other. At this time, the rotation lock falls under the action of the torsion spring. Therefore, the engagement part again falls into the recess section of the locking part, i.e., the rotation lock part is reconnected to the paper advancing member.

At this time, the linkage apparatus for the printer provided in the embodiments of the present application resets to the initial state.

The linkage apparatus provided in the embodiments of the present application has a simple structure, so that a customer can conveniently perform paper removal and paper placement operations of the printer just by opening and closing the door assembly. In this process, there is no need to additionally use an auxiliary assembly. Therefore, the risk of a malfunction such as paper jam caused by a foreign object being caught in the printer can be avoided.

It should be noted that, although only the structure and function of the linkage apparatus for a printer related to the present application have been described above, the linkage apparatus for a printer may further include other components and have other functions. For details, reference may be made to related technologies, and details are not described further herein.

In daily life, there are many cases where, for example, small-sized bill or document printers need to be used, and some small-sized printers have quite small openings. In some other cases, sharp components or parts are disposed at openings of the small-sized printers. By means of the linkage apparatus provided in the embodiments of the present application, even when using a printer having a narrow opening, such as an electrocardiograph report printer, there is no need to stick the hand into the opening of the printer, which is more convenient for a user to operate. The danger of finger scratching can also be avoided during use of a printer provided with a sharp component or part.

The embodiments of the present application further provide a printer, including the linkage apparatus described in the foregoing embodiments.

In the above embodiments, the structure of the linkage apparatus is described in detail, the content of which is incorporated herein, and will not described again here. For other configurations and functions of the printer, reference may be made to the related art, and details are not described herein.

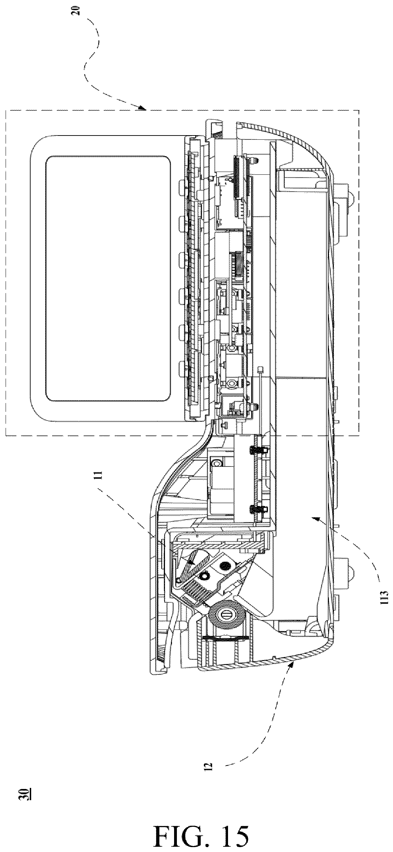

The embodiments of the present application further provide a medical apparatus, including the printer described in the foregoing embodiments. The medical apparatus includes, for example, an electrocardiograph machine. A schematic description is provided below with reference to FIG. 15.

FIG. 15 is a schematic view of an electrocardiograph machine 30 equipped with the linkage apparatus according to the embodiments of the present application. As shown in FIG. 15, the electrocardiograph machine 30 includes an electrocardiograph measurement component 20 and a printer, and the electrocardiograph measurement component 20 and the printer can be integrally formed by means of the same casing. The printer is equipped with the linkage apparatus provided in the embodiments of the present application. A paper compartment assembly 11, a door assembly 12, and a paper tray 113 used to accommodate paper (not shown) can be mounted on the printer of the electrocardiograph machine 30 by the means shown in FIG. 15.

Some existing electrocardiograph machines (for example, the integrated electrocardiograph machine 30 shown in FIG. 15) are required to be small and lightweight, due to the need for portability, and as a result, the overall volume of the printer and the area of the side opening through which paper is discharged are accordingly reduced. By means of installing the linkage apparatus according to the embodiments of the present application, operation processes such as printing, paper removal, and paper placement can be performed safely and easily even when the area of the side opening of the printer is small. The structure of the linkage apparatus is simple, and ease of installation and use are improved, thereby improving the user experience.

For the other components and functions of the electrocardiograph machine and the integrated printer thereof, reference may be made to any related technologies in the art. For example, the electrocardiograph measurement component 20 may include: any circuit and sensor needed for measuring an electrocardiographic signal, a memory having an electrocardiographic measurement algorithm stored thereon, a display for displaying an electrocardiographic measurement result, and a battery for providing electric power necessary for measuring, calculating and displaying functions, or the like. Further, the printer includes, in addition to the linkage apparatus shown in FIG. 15, any other components necessary for performing the printing function. The foregoing will not be described any further. In addition, although the embodiment of FIG. 15 is illustrated by an example of an electrocardiograph machine, the present application is not so limited, and other types of medical apparatuses, such as integrated medical apparatuses, are equally applicable to any of the above embodiments of the present application. The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

The embodiments of the present application are described above with reference to specific implementations. However, it should be clear to those skilled in the art that such description is merely illustrative and is not intended to limit the scope of protection of the embodiments of the present application. Various variations and modifications could be made by those skilled in the art according to the spirit and principle of the embodiments of the present application, and these variations and modifications also fall within the scope of the embodiments of the present application.

Preferred implementations of the embodiments of the present application are described above with reference to the accompanying drawings. Many features and advantages of the implementations are clear according to the detailed description, and therefore the appended claims are intended to cover all these features and advantages that fall within the true spirit and scope of these implementations. In addition, because many modifications and changes could be easily conceived of by those skilled in the art, implementations of the embodiments of the present application are not limited to the illustrated and described precise structures and operations, but can cover all appropriate modifications and equivalents that fall within the scope of the implementations.

## Claims

1. A linkage apparatus for a printer, **characterized in that** the linkage apparatus comprises:
a paper compartment assembly, provided with a side opening, a base, a paper tray located in the base, and a paper advancing member disposed opposite the side opening, the end of the paper advancing member opposite the side opening being provided with an advancing plate extending upward;
a door assembly, movably connected to the paper compartment assembly and capable of closing or opening the side opening; and
a transmission mechanism, one side of which is connected to the door assembly, the transmission mechanism driving the paper advancing member to move toward the side opening along with opening of the door assembly, causing the paper advancing member to, by means of the advancing plate, advance paper in the paper tray to move toward the door assembly.

2. The linkage apparatus according to claim 1, wherein the transmission mechanism comprises:
a gear transmission part, disposed in the base and provided with a gear set, a propeller shaft, and a rack linked to the gear set by means of the propeller shaft,
wherein at least one gear in the gear set is connected to the door assembly, and, along with opening and closing movements of the door assembly, the gear set drives the propeller shaft to rotate, thus driving the rack to move in the base, the rack being linked with the paper advancing member, and when the rack moves in the base, the paper advancing member accordingly moving in the base.

3. The linkage apparatus according to claim 2, wherein one end and the other end of the propeller shaft are provided with a first gear and a second gear, respectively, the first gear is connected to the gear set, and the second gear is connected to the rack.

4. The linkage apparatus according to claim 3, wherein the rack is provided with a step section, an extending section, and a connecting section,
wherein the step section meshes with the propeller shaft, the extending section extends from one end of the step section in a direction toward the paper advancing member, the connecting section extends from one end of the extending section in a direction toward the paper advancing member and is connected to the paper advancing member, and the width of the connecting section is less than that of the extending section.

5. The linkage apparatus according to claim 1, wherein the linkage apparatus further comprises a rotation lock part disposed in the base, one end of which is connected to the transmission mechanism, and the other end of which is connected to the paper advancing member so as to be openable and closable, the transmission mechanism, by means of the rotation lock part, driving the paper advancing member to move.

6. The linkage apparatus according to claim 5, wherein the rotation lock part is provided with: a shaft, a rotation arm, a torsion spring and a rotation lock; and
the rotation arm, the torsion spring and the rotation lock are fastened to the other side of the transmission mechanism by means of the shaft, and are rotatable about the shaft, so as to implement rotatable connection with the transmission mechanism.

7. The linkage apparatus according to claim 6, wherein the paper advancing member is further provided with a locking part, and a paper receiving part connected to the locking part, and the advancing plate protrudes upward from the paper receiving part; and
the rotation lock engages with the locking part, so as to connect the rotation lock part and the paper advancing member.

8. The linkage apparatus according to claim 7, wherein
the rotation lock is provided with an engagement part protruding downward from an extending end thereof;
the locking part is provided with a recess section recessed downward, and the engagement part engages with the recess section; and
an extending end of the locking part and an extending end of the engagement part of the rotation lock each have slopes whose shapes fit together.

9. The linkage apparatus according to claim 6, wherein the linkage apparatus further comprises a boss provided on the base; the rotation arm of the rotation lock part, after moving a particular distance in a direction toward the side opening, is capable of abutting against the boss and rotating, so as to release the engagement between the rotation lock and the locking part.

10. The linkage apparatus according to claim 9, wherein a hollow structure is provided between the transmission mechanism and the base, and the boss is accommodated in the hollow structure.

11. The linkage apparatus according to claim 9, wherein the linkage apparatus further comprises a limiting part, the limiting part is disposed at an end of the side opening of the base, and the limiting part is capable of preventing the door assembly from being opened farther when the door assembly is opened a particular amount.

12. The linkage apparatus according to claim 1, wherein
the paper tray comprises: a top cover and a bottom cover; and
the bottom cover is disposed on the base, and a space for accommodating paper is formed between the top cover and the bottom cover.

13. The linkage apparatus according to claim 12, wherein
the bottom cover is provided with an opening, and the opening has a shape matching that of the advancing plate of the paper advancing member, enabling the advancing plate of the paper advancing member to move in the opening.

14. A printer, **characterized in that** the printer comprises the linkage apparatus described in any one of claims 1 to 13.

15. A medical apparatus, **characterized in that** the medical apparatus comprises the printer described in claim 14.
